# EUROPEAN PATENT APPLICATION

(11) **EP 4 585 151 A1**
(43) Date of publication of application: **16.07.2025**
(21) Application number: 24150978.5
(22) Date of filing: 09.01.2024
(51) Int. Cl.: A61B 5/28, A61B 5/055, A61B 5/268, A61B 5/274

(54) **ELECTROCARDIOGRAM INTERFACING**

(71) Applicant: Koninklijke Philips N.V., 5656 AG Eindhoven (NL)
(72) Inventor: KOSTAKIS, Dimitri George, 5656 AG Eindhoven (NL)
(74) Representative: Philips Intellectual Property & Standards

(57) **Abstract**

An electrocardiogram cable includes an electrocardiogram cable body and an electrocardiogram cable interface configured to detachably fit into an electrocardiogram electrode interface from one direction. An electrocardiogram electrode includes an electrocardiogram electrode main body and an electrocardiogram electrode interface configured to detachably accept an electrocardiogram cable interface from one direction. The electrocardiogram cable and the electrocardiogram electrode are configured for use in magnetic resonance imaging.

## Description

### FIELD OF THE INVENTION

The invention relates to an electrocardiogram cable, an electrocardiogram electrode and a method of connecting an electrocardiogram cable to an electrocardiogram electrode.

### BACKGROUND OF THE INVENTION

Electrocardiogram (ECG) is a patient measurement parameter frequently used in measurement devices to measure heart rate and amplitude. The measurement device receives electrical signals sent from the patient. An electrode accessory worn by the patient is attached to a conductive cable and the conductive cable is attached to the measurement device. Conductive cables for electrocardiogram measurements are typically designed with conductive copper wiring in the majority of the medical facility settings such as hospital settings. The connection between the conductive cable and the electrode accessory is typically a metal contact.

As a result of the concerns for magnetic resonance (MR) environments, methods of connecting electrode accessories should be tailored to the MR environments instead of other environments in medical facilities. Typical conductive cables and electrode accessories used for ECG measurements are inappropriate for a magnetic resonance (MR) environment. For MR environments, conductive copper wiring is rarely used due to the risk of creating magnetic attraction and the resulting RF heating effect. Instead, for conductive cables used for ECG measurements in MR environments, high impedance wiring is used to transmit the small voltages coming from the electrode accessories to the measurement device. Additionally, the material used for the electrode accessory and cable contact is a conductive plastic, often a carbon filled acrylonitrile-butadiene-styrene (ABS), though conductive plastics used for electrode accessories and cable contacts are not particularly limited to ABS.

A few characteristics required for equipment used in the MR environment include cleanability, material composition, and reliability.

The MR environment requires increased levels of cleaning. For example, the patient throughput in the MR environment is higher than other parts of the hospital such that materials used in the MR environment have higher cleaning requirements. Typical mechanisms for ECG connection are complex and have geometry with numerous dirt traps which are difficult to clean and disinfect.

Additionally, fastening mechanisms in the MR environment cannot include conventional metal contact connections that snap together to avoid creating magnetic attraction to the MR scanner or heating effects. Conductive plastic materials are used instead of metals in the MR environment for connection between conductive cable and electrode accessories.

However, snapping and unsnapping of the fastening mechanisms made of conductive plastic materials causes wear and other reliability issues over time. For example, fastening mechanisms at the ends of some conductive cables sometimes require a user to press a snap mechanism open before applying to the electrode accessory, and this movement presents a high reliability risk.

Conventional electrode accessories have an interface formed of a post and the cables have an interface that snaps onto the post. Resultingly, the complexity in the interface between an electrode accessory and a conductive cable is on the cable side. As an example, leads on an electrode accessory may require opening, and then the fastening mechanisms at the ends of the conductive cables are snapped onto the open lead.

### SUMMARY OF THE INVENTION

The invention is defined by the independent claims. Advantageous embodiments are defined in the dependent claims.

According to an aspect of the present disclosure, an electrocardiogram cable includes an electrocardiogram cable body; and an electrocardiogram cable interface. The electrocardiogram cable interface is configured to detachably fit into an electrocardiogram electrode interface from one direction. The electrocardiogram cable is configured for use in magnetic resonance imaging.

According to another aspect of the present disclosure, an electrocardiogram electrode includes an electrocardiogram electrode main body; and an electrocardiogram electrode interface. The electrocardiogram electrode interface is configured to detachably accept an electrocardiogram cable interface from one direction. The electrocardiogram electrode is configured for use in magnetic resonance imaging.

According to another aspect of the present disclosure, a method for connecting an electrocardiogram cable to an electrocardiogram electrode includes detachably accepting, by an electrocardiogram electrode interface, an electrocardiogram cable interface from one direction. The method also includes detachably locking, by the electrocardiogram electrode interface, the electrocardiogram cable interface into place in a plurality of directions. The electrocardiogram cable and the electrocardiogram electrode are configured for use in magnetic resonance imaging.

These and other aspects of the invention will be apparent from and elucidated with reference to the embodiments described hereinafter.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
FIG. 1 illustrates a system for electrocardiogram interfacing, in accordance with a representative embodiment.
FIG. 2 illustrates an electrocardiogram electrode and an electrocardiogram cable for electrocardiogram interfacing, in accordance with a representative embodiment.
FIG. 3 illustrates an electrocardiogram electrode and an electrocardiogram cable for electrocardiogram interfacing, in accordance with a representative embodiment.
FIG. 4 illustrates an electrocardiogram electrode and an electrocardiogram cable for electrocardiogram interfacing, in accordance with a representative embodiment.
FIG. 5 illustrates a method for electrocardiogram interfacing, in accordance with a representative embodiment.
FIG. 6 illustrates a computer system, on which a method for processing and displaying signals from electrocardiogram electrodes is implemented, in accordance with another representative embodiment.

### DESCRIPTION OF EMBODIMENTS

In the following detailed description, for the purposes of explanation and not limitation, representative embodiments disclosing specific details are set forth in order to provide a thorough understanding of embodiments according to the present teachings. However, other embodiments consistent with the present disclosure that depart from specific details disclosed herein remain within the scope of the appended claims. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. Definitions and explanations for terms herein are in addition to the technical and scientific meanings of the terms as commonly understood and accepted in the technical field of the present teachings.

It will be understood that, although the terms first, second, third etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

As used in the specification and appended claims, the singular forms of terms 'a', 'an' and 'the' are intended to include both singular and plural forms, unless the context clearly dictates otherwise. Additionally, the terms "comprises", and/or "comprising," and/or similar terms when used in this specification, specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to", "coupled to", or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below.

As described herein, configurations for electrocardiogram cables and electrocardiogram electrodes can be improved for use in a magnetic resonance imaging context. The improvements described herein make the combination of electrocardiogram cables and electrocardiogram electrodes easier to clean, more reliable, and usable in magnetic resonance imaging environments. A method of connection between an electrocardiogram cable and an accessory electrode suitable for magnetic resonance environments provides optimized cleanability, reliability, and material selection.

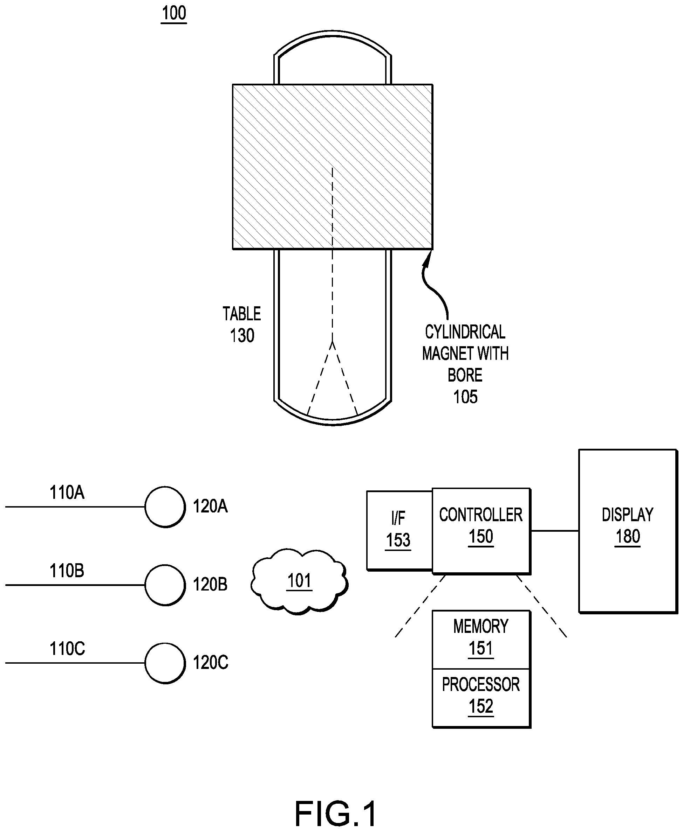

FIG. 1 illustrates a system 100 for electrocardiogram interfacing, in accordance with a representative embodiment.

The system 100 in FIG. 1 is a system for electrocardiogram interfacing and includes components that may be provided together or separately. The system 100 includes magnetic resonance imaging system with a cylindrical magnet with bore 105 and a table 130, a first electrocardiogram cable 110A, a first electrocardiogram electrode 120A, a second electrocardiogram cable 110B, a second electrocardiogram electrode 120B, a third electrocardiogram cable 110C, a third electrocardiogram electrode 120C, a network 101, a controller 150, and a display 180. Each electrocardiogram cable in FIG. 1 may comprise conductive plastic and may also be reusable. Each electrocardiogram electrode in FIG. 1 may also comprise conductive plastic and may also be disposable. Additionally, each electrocardiogram cable in FIG. 1 may comprise a post as an electrocardiogram cable interface, and each electrocardiogram electrode in FIG. 1 may comprise a snap that snaps onto the post.

The network 101 may comprise a wireless local area network (LAN) such as a Wi-Fi network.

The combinations of the first electrocardiogram cable 110A and first electrocardiogram electrode 120A, the second electrocardiogram cable 110B and second electrocardiogram electrode 120B, and the third electrocardiogram cable 110C and third electrocardiogram electrode 120C are configured for use on a patient on the table 130 when the patient is undergoing magnetic resonance imaging in the bore 105. The components of the first electrocardiogram cable 110A, the first electrocardiogram electrode 120A, the second electrocardiogram cable 110B, the second electrocardiogram electrode 120B, the third electrocardiogram cable 110C and the third electrocardiogram electrode 120C are made of materials which are deemed safe in the environment of magnetic resonance imaging. Although three sets of electrocardiogram cables and electrocardiogram electrodes are shown in FIG. 1, more or fewer than three sets of electrocardiogram cables and electrocardiogram electrodes may be used in the system 100.

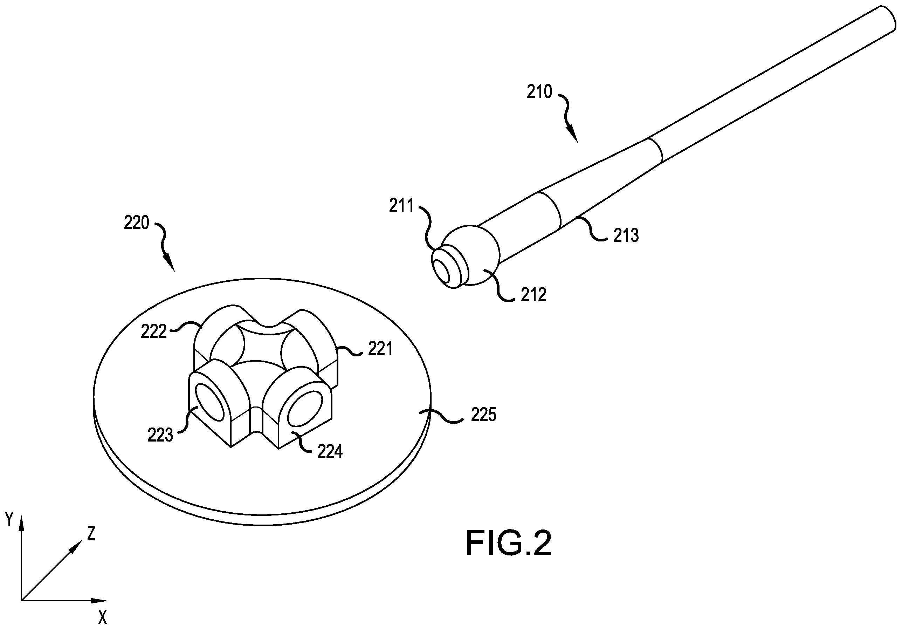

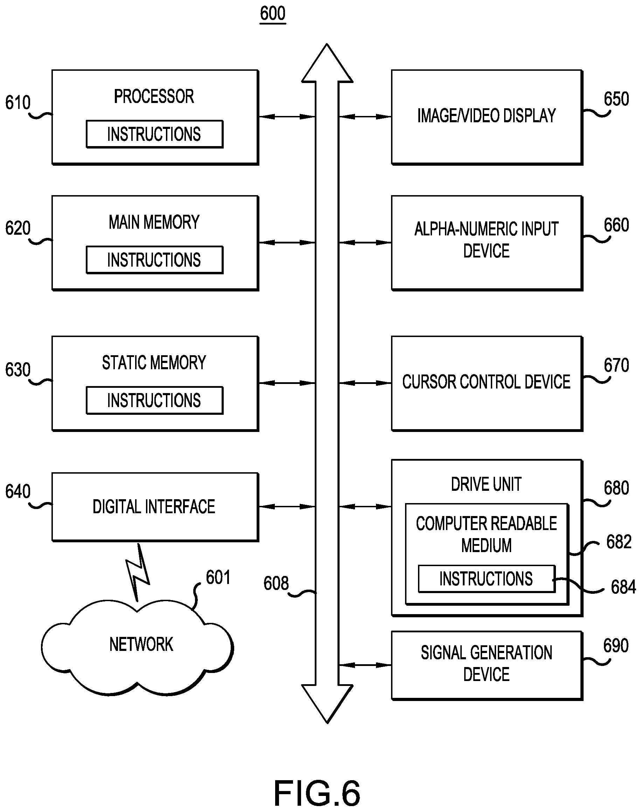

The controller 150 includes at least a memory 151 that stores instructions, a processor 152 that executes the instructions, and an interface 153. An example of a device that includes the controller 150 is a MR compatible medical monitor such as a patient monitor. The controller 150 may be implemented in a computer as depicted in FIG. 6, though a controller 150 may include more elements than in FIG. 1 and fewer elements than the computer in FIG. 6. The interface 153 may comprise an interface connected directly by wire or indirectly and wirelessly to the first electrocardiogram electrode 120A, the second electrocardiogram electrode 120B and the third electrocardiogram electrode 120C. The interface 153 may include ports, wireless antennas, or other types of receiver circuitry that connect the controller 150 to the electrocardiogram electrodes. The controller 150 may also include other types of interfaces such as user interfaces including buttons, keys, a mouse, a microphone, a speaker, a display separate from the display 180, or other elements that users can use to interact with the controller 150 such as to enter instructions and receive output. Examples of the combinations of electrocardiogram cables and the electrocardiogram electrodes are shown in and explained with respect to FIG. 2, FIG. 3 and FIG. 4.

The display 180 may be local to the controller 150 or may be remotely connected to the controller 150. The display 180 may be connected to the controller 150 via a local wired interface such as an Ethernet cable or via a local wireless interface such as a Wi-Fi connection. The display 180 may be interfaced with other user input devices by which users can input instructions, including mice, keyboards, thumbwheels and so on. The display 180 may be a monitor such as a computer monitor, a display on a mobile device, an augmented reality display, a television, an electronic whiteboard, or another screen configured to display electronic imagery. The display 180 may also include one or more input interface(s) such as those noted above that may connect to other elements or components, as well as an interactive touch screen configured to display prompts to users and collect touch input from users. The display 180 may be configured to display waveforms of readings from the electrocardiogram electrodes received at the controller 150 via the electrocardiogram cables.

The controller 150 may perform operations to process signals from the electrocardiogram electrodes, such as to result in visualizations of electrocardiogram waveforms on the display 180. For example, the controller 150 may indirectly control operations such as by generating and transmitting content to be displayed on the display 180. The controller 150 may directly control other operations such as logical operations performed by the processor 152 executing instructions from the memory 151 based on input received from the electrocardiogram electrodes and/or users via the interface 153 and any other interface. For example, the controller 150 may create digital data used to generate the electrocardiogram waveforms on the display 180.

The combinations of electrocardiogram cables and electrocardiogram electrodes in FIG. 1 shift the mechanisms of interfacing by shifting the complexity from the reusable electrocardiogram cables to the disposable electrocardiogram electrodes. As a result, cleanability of the electrocardiogram cable is optimized as the geometry of the reusable electrocardiogram cable is simplified with fewer dirt trap surfaces. The reusable electrocardiogram cables in FIG. 1 may comprise a post interface, so that the disposable electrode has a more complex snap interface. This moves the reliability issues for a multi-use cable to the single use disposable accessory. As a result, the electrocardiogram cable interfaces are easier to clan and have minimal dirt traps.

This updated geometry can now be designed in combination with increased opportunities for material selection of other conductive plastics and non-magnetic conductive materials tailored for the use model of the cable.

As described herein, each electrocardiogram cable in one of the combinations in FIG. 1 includes an electrocardiogram cable body, and an electrocardiogram cable interface. The electrocardiogram cable interface is configured to detachably fit into an electrocardiogram electrode interface of the electrocardiogram electrode in the combination from one direction. The electrocardiogram cable in each combination is configured for use in magnetic resonance imaging. As also described herein, each electrocardiogram electrode in one of the combinations in FIG. 1 includes an electrocardiogram electrode main body, and an electrocardiogram electrode interface. The electrocardiogram electrode interface is configured to detachably accept an electrocardiogram cable interface of the electrocardiogram cable in the combination from one direction. The electrocardiogram electrode is configured for use in magnetic resonance imaging. Electrocardiogram cables and electrocardiogram electrodes are described further with respect to FIG. 2, FIG. 3 and FIG. 4.

FIG. 2 illustrates an electrocardiogram electrode and an electrocardiogram cable for electrocardiogram interfacing, in accordance with a representative embodiment.

The electrocardiogram cable 210 in FIG. 2 includes an electrocardiogram cable interface and an electrocardiogram cable body 213. The electrocardiogram cable interface in FIG. 2 includes an insertion portion 211 and a locking portion 212. The electrocardiogram cable interface in FIG. 2 is configured to detachably fit into an electrocardiogram electrode interface from one direction of the electrocardiogram electrode 220 in FIG. 2. The electrocardiogram cable 210 is configured for use in magnetic resonance imaging.

The electrocardiogram electrode 220 in FIG. 2 includes an electrocardiogram electrode main body 225 and four separate electrocardiogram electrode interfaces configured to detachably accept an electrocardiogram cable interface from one direction. The electrocardiogram electrode 220 is configured for use in magnetic resonance imaging as in FIG. 1. The four electrocardiogram electrode interfaces in FIG. 2 include a first electrocardiogram electrode interface 221, a second electrocardiogram electrode interface 222, a third electrocardiogram electrode interface 223, and a fourth electrocardiogram electrode interface 224. The four electrocardiogram electrode interfaces in FIG. 2 are arranged in four coplanar directions which are spaced 90 degrees or 180 degrees from one another. The coplanar directions may be formed in an XZ plane as shown. In FIG. 2, the four electrocardiogram electrode interfaces may be arranged above the electrocardiogram electrode main body 225 in the Y dimension as shown. The directions X, Y and Z in FIG. 2 are each perpendicular from one another. The four electrocardiogram electrode interfaces in FIG. 2 may each be fixed to the electrocardiogram electrode main body 225 and may each be fixed to each of the two adjacent electrocardiogram interfaces.

In FIG. 2, the electrocardiogram cable interface with the insertion portion 211 and the locking portion 212 is configured to be inserted into one of the four separate electrocardiogram electrode interfaces from one direction. For example, the insertion portion 211 and the locking portion 212 may be configured to be inserted through one of the four separate electrocardiogram electrode interfaces partially or fully. The one direction in FIG. 2 may be parallel to the electrocardiogram electrode main body 225 in FIG. 2. The locking portion 212 may comprise a relatively soft material that can be compressed to fit into an electrocardiogram electrode interface and then expand once inside. The insertion portion 211 serves as a lead for the locking portion 212.

In FIG. 2, the four electrocardiogram electrode interfaces are shown and described to be coplanar. However, as few as one and more than four electrocardiogram electrode interfaces may be provided on an electrocardiogram electrode main body 225. Additionally, there is not any particular requirement for multiple electrocardiogram electrode interfaces to be coplanar, though making them so may make it easier to manufacture the electrocardiogram electrode 220. Additionally, the four electrocardiogram electrode interfaces may include passive devices used to transmit electrical signals from the patient to the monitor or other electronics device connected to the other end of the respective cables.

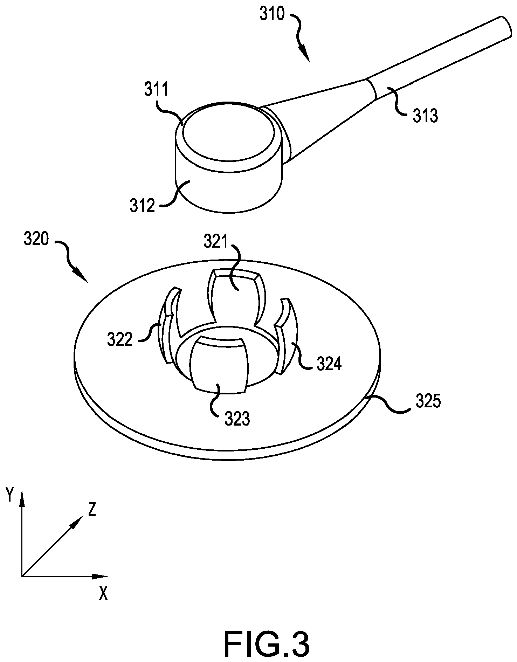

FIG.3 illustrates an electrocardiogram electrode and an electrocardiogram cable for electrocardiogram interfacing, in accordance with a representative embodiment.

The electrocardiogram cable 310 in FIG. 3 includes an electrocardiogram cable interface and an electrocardiogram cable body 313. The electrocardiogram cable interface in FIG. 3 includes a trailing portion 311 and a locking portion 312. The electrocardiogram cable interface in FIG. 3 is configured to detachably fit into an electrocardiogram electrode interface from one direction of the electrocardiogram electrode 320 in FIG. 3. The electrocardiogram cable 310 is configured for use in magnetic resonance imaging.

The electrocardiogram electrode 320 in FIG. 3 includes an electrocardiogram electrode main body 325 and a single instance of an electrocardiogram electrode interface configured to detachably accept an electrocardiogram cable interface from one direction. The single instance of the electrocardiogram electrode interface in FIG. 3 includes four locking members configured to accept and then lock the electrocardiogram cable interface in FIG. 3. The electrocardiogram electrode 320 is configured for use in magnetic resonance imaging as in FIG. 1. The four locking members include a first locking member 321, a second locking member 322, a third locking member 323, and a fourth locking member 324. The four locking members in FIG. 3 may be arranged to be substantially perpendicular to the electrocardiogram electrode main body 325, and may be spaced 90 degrees or 180 degrees from one another. The coplanar directions may be formed in an XZ plane as shown. In FIG. 3, the four insertion members are arranged above the electrocardiogram electrode main body 325 in the Y dimension as shown. The directions X, Y and Z in FIG. 3 are each perpendicular from one another.

The four locking members in FIG. 3 may be fixed to the electrocardiogram electrode main body 325 at the bottom, and adjustable relative forward (towards a center of the electrocardiogram electrode main body 325) and backward (away from the center of the electrocardiogram electrode main body 325) at the top.

In FIG. 3, the electrocardiogram cable interface with the trailing portion 311 and the locking portion 312 is configured to be inserted into one instance of the electrocardiogram electrode interface from one direction, i.e., from above. The one direction in FIG. 3 is optionally perpendicular to the electrocardiogram electrode main body 325. The locking portion 312 may comprise a relatively soft material that can be compressed to fit into an electrocardiogram electrode interface and then expand once inside. The trailing portion 311 serves as a trailer for the locking portion 312, and may accommodate the uppermost portions of the four locking members to lock the electrocardiogram cable interface in place.

In FIG. 3, four locking members are shown and the four locking members are shown and described to be attached to the same surface of the electrocardiogram electrode main body 325. However, as few as two and more than four locking members may be provided on an electrocardiogram electrode main body 325. Additionally, there is not any particular requirement for multiple locking members to be sourced from the same surface of an electrocardiogram electrode main body 325, though again making them so may make it easier to manufacture the electrocardiogram electrode 320.

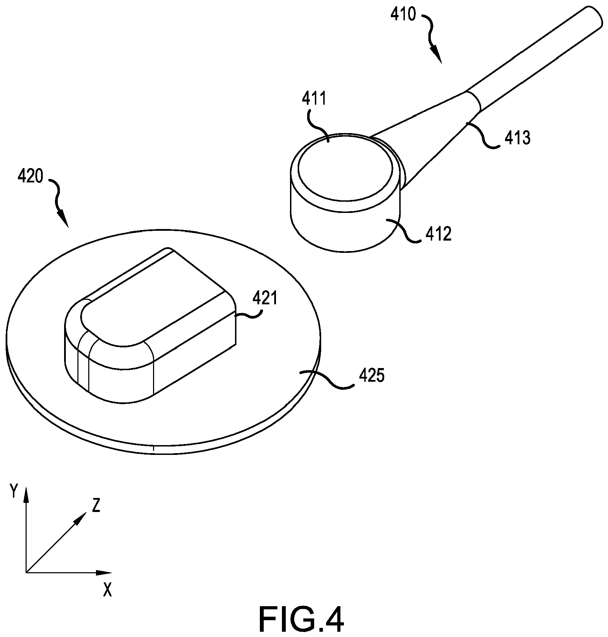

FIG. 4 illustrates an electrocardiogram electrode and an electrocardiogram cable for electrocardiogram interfacing, in accordance with a representative embodiment.

The electrocardiogram cable 410 in FIG. 4 includes an electrocardiogram cable interface and an electrocardiogram cable body 413. The electrocardiogram cable interface in FIG. 4 includes an upper portion 411 and a locking portion 412. The electrocardiogram cable interface in FIG. 4 is configured to detachably fit into an electrocardiogram electrode interface from one direction of the electrocardiogram electrode 420 in FIG. 4. The electrocardiogram cable 410 is configured for use in magnetic resonance imaging.

The electrocardiogram electrode 420 in FIG. 4 includes an electrocardiogram electrode main body 425 and a single instance of an electrocardiogram electrode interface configured to detachably accept an electrocardiogram cable interface from one direction. The single instance of the electrocardiogram electrode interface in FIG. 4 includes a single instance of a locking member 421 configured to accept and then lock the electrocardiogram cable interface in FIG. 4. The electrocardiogram electrode 420 is configured for use in magnetic resonance imaging as in FIG. 1. The single instance of a locking member 421 in FIG. 4 is disposed on and may be parallel to an upper surface of the electrocardiogram electrode main body 425, so that the electrocardiogram cable interface in FIG. 4 may be inserted from one direction which is parallel to the upper surface of the electrocardiogram electrode main body 425 (i.e., from a single lateral direction). The upper portion 411 has a smaller diameter than the locking portion 412, so that the electrocardiogram cable interface in FIG. 4 fits into the electrocardiogram electrode interface similar to the way a key fits into a keyhole.

In FIG. 4, the electrocardiogram cable interface with the upper portion 411 and the locking portion 412 is configured to be inserted into the one instance of the electrocardiogram electrode interface from one direction, i.e., from the side. The one direction in FIG. 4 is optionally parallel to the electrocardiogram electrode main body 425 in FIG. 4. The locking portion 412 may comprise a relatively soft material that can be compressed to fit into an electrocardiogram electrode interface and then expand once inside.

In FIG. 4, a single instance of the locking member 421 is shown. However, more than one instance of a locking member 421 may be provided on an electrocardiogram electrode main body 425.

The combinations in FIG. 2, FIG. 3 and FIG. 4 show examples of the geometry for combinations of electrocardiogram cable interfaces and electrocardiogram electrode interfaces. With these types of geometry, reliability, wear and cleaning issues are reduced at least for the electrocardiogram cables and electrocardiogram cable interfaces, if not also for the electrocardiogram electrode and electrocardiogram electrode interfaces. The teachings herein are not limited to these geometries for the electrocardiogram cable and the electrocardiogram electrode. In any geometry consistent with the teachings herein, however, the burden of reliability and cleanability issues is moved to the electrocardiogram electrode insofar as electrocardiogram electrodes are typically disposable.

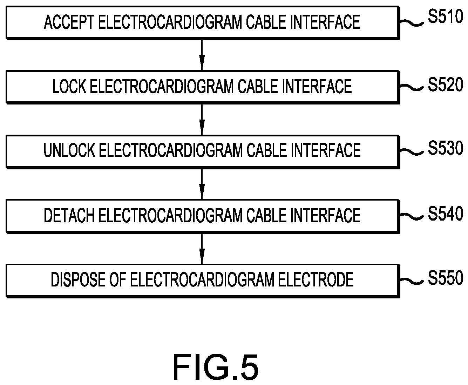

FIG. 5 illustrates a method for electrocardiogram interfacing, in accordance with a representative embodiment.

At S510, an electrocardiogram cable interface is accepted. The electrocardiogram cable interface may be accepted by any one of the electrocardiogram electrode interfaces in FIG. 2, FIG. 3 or FIG. 4.

At S520, the electrocardiogram cable interface is locked. The electrocardiogram cable interface may be locked automatically by squeezing into one of the electrocardiogram electrode interfaces in FIG. 2 and then expanding. Relative to FIG. 3, the electrocardiogram cable interface may be locked automatically by squeezing into the four locking portions of the electrocardiogram electrode interface. Alternatively, relative to FIG. 3, one or more of the four locking portions may be flexible or otherwise adjustable to allow the electrocardiogram cable interface into the electrocardiogram electrode interface, and then adjust or be adjusted to lock the electrocardiogram cable interface into place. Relative to FIG. 4, the electrocardiogram cable interface may be locked into place by friction or by locking members that close behind the upper portion 411 and the locking portion 412.

At S530, the electrocardiogram cable interface is unlocked. For example, after use the electrocardiogram cable interface may be unlocked by applying force to pull the electrocardiogram cable interface from the electrocardiogram electrode interface. Alternatively, one or more locking members as in FIG. 3 may be loosened or otherwise adjusted to allow the electrocardiogram cable interface to be detached from the electrocardiogram electrode interface.

At S540, the electrocardiogram cable interface is detached from the electrocardiogram electrode interface, such as by pulling the two interfaces apart.

At S550, the electrocardiogram electrode is disposed of, such as by placing the electrocardiogram electrode into a trash receptacle. The electrocardiogram electrode described herein may be disposable, and by placing complexity of a receiving component on the electrocardiogram electrode, burdens of cleaning or otherwise maintaining complex components may be minimized.

FIG. 6 illustrates a computer system, on which a method for electrocardiogram interfacing is implemented, in accordance with another representative embodiment.

Referring to FIG.6, the computer system 600 includes a set of software instructions that can be executed to cause the computer system 600 to perform computer-based functions disclosed herein. The computer system 600 may operate as a standalone device or may be connected, for example, using a network 601, to other computer systems or peripheral devices. In embodiments, a computer system 600 performs logical processing based on digital signals received via an analog-to-digital converter.

In a networked deployment, the computer system 600 may operate as a client user computer in a server-client user network environment. The computer system 600 can also be implemented as or incorporated into various devices, such as a patient monitor or other medical device that includes a controller, a stationary computer, a mobile computer, a personal computer (PC), a laptop computer, a tablet computer, or any other machine capable of executing a set of software instructions (sequential or otherwise) that specify actions to be taken by that machine. The computer system 600 can be incorporated as or in a device that in turn is in an integrated system that includes additional devices. In an embodiment, the computer system 600 can be implemented using electronic devices that provide voice, video or data communication. Further, while the computer system 600 is illustrated in the singular, the term "system" shall also be taken to include any collection of systems or sub-systems that individually or jointly execute a set, or multiple sets, of software instructions to perform one or more computer functions.

As illustrated in FIG. 6, the computer system 600 includes a processor 610. The processor 610 may be considered a representative example of a processor of a controller and executes instructions to implement some processes described herein. The processor 610 is tangible and non-transitory. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The processor 610 is an article of manufacture and/or a machine component. The processor 610 is configured to execute software instructions to perform functions as described in the various embodiments herein. The processor 610 may be a general-purpose processor or may be part of an application specific integrated circuit (ASIC). The processor 610 may also be a microprocessor, a microcomputer, a processor chip, a controller, a microcontroller, a digital signal processor (DSP), a state machine, or a programmable logic device. The processor 610 may also be a logical circuit, including a programmable gate array (PGA), such as a field programmable gate array (FPGA), or another type of circuit that includes discrete gate and/or transistor logic. The processor 610 may be a central processing unit (CPU), a graphics processing unit (GPU), or both. Additionally, any processor described herein may include multiple processors, parallel processors, or both. Multiple processors may be included in, or coupled to, a single device or multiple devices.

The term "processor" as used herein encompasses an electronic component able to execute a program or machine executable instruction. References to a computing device comprising "a processor" should be interpreted to include more than one processor or processing core, as in a multi-core processor. A processor may also refer to a collection of processors within a single computer system or distributed among multiple computer systems. The term computing device should also be interpreted to include a collection or network of computing devices each including a processor or processors. Programs have software instructions performed by one or multiple processors that may be within the same computing device or which may be distributed across multiple computing devices.

The computer system 600 further includes a main memory 620 and a static memory 630, where memories in the computer system 600 communicate with each other and the processor 610 via a bus 608. Either or both of the main memory 620 and the static memory 630 may be considered representative examples of a memory of a controller, and store instructions used to implement some processes described herein. Memories described herein are tangible storage mediums for storing data and executable software instructions and are non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The main memory 620 and the static memory 630 are articles of manufacture and/or machine components. The main memory 620 and the static memory 630 are computer-readable mediums from which data and executable software instructions can be read by a computer (e.g., the processor 610). Each of the main memory 620 and the static memory 630 may be implemented as one or more of random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM), digital versatile disk (DVD), floppy disk, blu-ray disk, or any other form of storage medium known in the art. The memories may be volatile or non-volatile, secure and/or encrypted, unsecure and/or unencrypted.

"Memory" is an example of a computer-readable storage medium. Computer memory is any memory which is directly accessible to a processor. Examples of computer memory include, but are not limited to RAM memory, registers, and register files. References to "computer memory" or "memory" should be interpreted as possibly being multiple memories. The memory may for instance be multiple memories within the same computer system. The memory may also be multiple memories distributed amongst multiple computer systems or computing devices.

As shown, the computer system 600 further includes a video display unit 650, such as a liquid crystal display (LCD), an organic light emitting diode (OLED), a flat panel display, a solid-state display, or a cathode ray tube (CRT), for example. Additionally, the computer system 600 includes an input device 660, such as a keyboard/virtual keyboard or touch-sensitive input screen or speech input with speech recognition, and a cursor control device 670, such as a mouse or touch-sensitive input screen or pad. The computer system 600 also optionally includes a disk drive unit 680, a signal generation device 690, such as a speaker or remote control, and/or a network interface device 640.

In an embodiment, as depicted in FIG. 6, the disk drive unit 680 includes a computer-readable medium 682 in which one or more sets of software instructions 684 (software) are embedded. The sets of software instructions 684 are read from the computer-readable medium 682 to be executed by the processor 610. Further, the software instructions 684, when executed by the processor 610, perform one or more steps of processes described herein. In an embodiment, the software instructions 684 reside all or in part within the main memory 620, the static memory 630 and/or the processor 610 during execution by the computer system 600. Further, the computer-readable medium 682 may include software instructions 684 or receive and execute software instructions 684 responsive to a propagated signal, so that a device connected to a network 601 communicates voice, video or data over the network 601. The software instructions 684 may be transmitted or received over the network 601 via the network interface device 640.

In an embodiment, dedicated hardware implementations, such as application-specific integrated circuits (ASICs), field programmable gate arrays (FPGAs), programmable logic arrays and other hardware components, are constructed to implement aspects of processes described herein. One or more embodiments described herein may implement functions using two or more specific interconnected hardware modules or devices with related control and data signals that can be communicated between and through the modules. Accordingly, the present disclosure encompasses software, firmware, and hardware implementations. Nothing in the present application should be interpreted as being implemented or implementable solely with software and not hardware such as a tangible non-transitory processor and/or memory.

Accordingly, electrocardiogram interfacing provides configurations for electrocardiogram cables and electrocardiogram electrodes that are improved for use in a magnetic resonance imaging context. The improvements described herein make the combination of electrocardiogram cables and electrocardiogram electrodes easier to clean, more reliable, and usable in magnetic resonance imaging environments. A method of connection between an electrocardiogram cable and an accessory electrode suitable for magnetic resonance environments provides optimized cleanability, reliability, and material selection.

Although electrocardiogram interfacing has been described with reference to several exemplary embodiments, it is understood that the words that have been used are words of description and illustration, rather than words of limitation.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true spirit and scope of the present disclosure. Thus, to the maximum extent allowed by law, the scope of the present disclosure is to be determined by the broadest permissible interpretation of the following claims and their equivalents and shall not be restricted or limited by the foregoing detailed description.

## Claims

1. An electrocardiogram cable (210), comprising:
an electrocardiogram cable body (213); and
an electrocardiogram cable (210) interface (153) configured to detachably fit into an electrocardiogram electrode (220) interface (153) from one direction, wherein the electrocardiogram cable (210) is configured for use in magnetic resonance imaging.

2. The electrocardiogram cable (210) of claim 1, wherein the electrocardiogram cable (210) interface (153) is configured to be detachably fit into the electrocardiogram electrode (220) interface (153) from a side of the electrocardiogram electrode (220) interface (153) and parallel to a main body of an electrode that includes the electrocardiogram electrode (220) interface (153).

3. The electrocardiogram cable (210) of claim 1, wherein the electrocardiogram cable (210) interface (153) is configured to be detachably fit into the electrocardiogram electrode (220) interface (153) from a side of the electrocardiogram electrode (220) interface (153) and perpendicular to a main body of an electrode that includes the electrocardiogram electrode (220) interface (153).

4. The electrocardiogram cable (210) of claim 1, wherein the electrocardiogram cable (210) comprises a conductive plastic.

5. The electrocardiogram cable (210) of claim 1, wherein the electrocardiogram electrode (220) is disposable.

6. The electrocardiogram cable (210) of claim 1, wherein the electrocardiogram cable (210) comprises a post and the electrocardiogram electrode (220) comprises a snap that snaps onto the post.

7. An electrocardiogram electrode (220), comprising:
an electrocardiogram electrode main body (225); and
an electrocardiogram electrode (220) interface (153) configured to detachably accept an electrocardiogram cable (210) interface (153) from one direction, wherein the electrocardiogram electrode (220) is configured for use in magnetic resonance imaging.

8. The electrocardiogram electrode (220) of claim 7, wherein the electrocardiogram electrode (220) interface (153) is configured to detachably accept the electrocardiogram cable (210) interface (153) from a side of the electrocardiogram electrode (220) interface (153) and parallel to the electrocardiogram electrode main body (225).

9. The electrocardiogram electrode (220) of claim 7, wherein the electrocardiogram electrode (220) interface (153) is configured to detachably accept the electrocardiogram cable (210) interface (153) from a side of the electrocardiogram electrode (220) interface (153) and perpendicular to the electrocardiogram electrode main body (225).

10. The electrocardiogram electrode (220) of claim 7, wherein the electrocardiogram electrode (220) comprises a conductive plastic.

11. The electrocardiogram electrode (220) of claim 7, wherein the electrocardiogram electrode (220) is disposable.

12. The electrocardiogram electrode (220) of claim 7, wherein the electrocardiogram cable (210) comprises a post and the electrocardiogram electrode (220) comprises a snap that snaps onto the post.

13. A method for connecting an electrocardiogram cable (210) to an electrocardiogram electrode (220), comprising:
detachably accepting, by an electrocardiogram electrode (220) interface (153), an electrocardiogram cable (210) interface (153) from one direction; and
detachably locking, by the electrocardiogram electrode (220) interface (153), the electrocardiogram cable (210) interface (153) into place in a plurality of directions, wherein the electrocardiogram cable (210) and the electrocardiogram electrode (220) are configured for use in magnetic resonance imaging.

14. The method of claim 13, wherein the electrocardiogram electrode (220) interface (153) is configured to detachably accept the electrocardiogram cable (210) interface (153) from a side of the electrocardiogram electrode (220) interface (153) and parallel to an electrocardiogram electrode main body (225).

15. The method of claim 13, wherein the electrocardiogram electrode (220) interface (153) is configured to detachably accept the electrocardiogram cable (210) interface (153) from a side of the electrocardiogram electrode (220) interface (153) and perpendicular to an electrocardiogram electrode main body (225).
